# FASCICULE DE BREVET EUROPEEN

(11) **EP 1 377 183 B1**
(45) Date de publication et mention de la délivrance du brevet: **09.01.2008**
(21) Numéro de dépôt: 02735451.3
(22) Date de dépôt: 10.04.2002
(51) Int. Cl.: A23L 1/308, A61K 36/00, A61K 9/52

(54) **PREPARATION A BASE DE CACTACEAE AYANT LA PROPRIETE DE FIXER LES GRAISSES, ET PROCEDE D'OBTENTION D'UNE TELLE PREPARATION**
CACTACEA ZUBEREITUNG MIT FETTBINDENDEN EIGENSCHAFTEN, SOWIE VERFAHREN IHRER HERSTELLUNG
CACTACEAE-BASED FORMULATION HAVING THE PROPERTY OF FIXING FATS, AND METHOD FOR OBTAINING SAME

(30) Priorité: 11.04.2001 FR 0104960
(43) Date de publication de la demande: 07.01.2004
(73) Titulaire: Bio Serae Laboratoires SA., 11150 Bram (FR)
(72) Inventeur: D'HUART, Jean-Baptiste, F-11000 Carcassonne (FR); DALLAS, Constantin, F-34500 Beziers (FR)
(74) Mandataire: Cabinet BARRE LAFORGUE & associés
(86) Numéro de dépôt international: PCT/FR2002/001254
(87) Numéro de publication internationale: WO 2002/082930

(56) Documents cités:
- WO-A-01/87078
- DATABASE WPI Section Ch, Week 198427 Derwent Publications Ltd., London, GB; Class D13, AN 1984-168191 XP002189939 -& JP 59 091856 A (IWASAKI S), 26 mai 1984 (1984-05-26) cité dans la demande
- DATABASE WPI Section Ch, Week 200056 Derwent Publications Ltd., London, GB; Class B04, AN 2000-588039 XP002189940 & CN 1 260 183 A (ZHEJIANG PROV PEOPLES HOSPITAL), 19 juillet 2000 (2000-07-19) cité dans la demande
- M. MUNOZ DE CHAVEZ ET AL.: "The Nopal: A Plant of Manifold Qualities" WORLD REVIEW OF NUTRITION AND DIETETICS., vol. 77, 1995, pages 109-134, XP001053507 KARGER, MUENCHEN., DE ISSN: 0084-2230
- PATENT ABSTRACTS OF JAPAN vol. 008, no. 030 (C-209), 8 février 1984 (1984-02-08) -& JP 58 194818 A (SHIYUUZOU IWASAKI), 12 novembre 1983 (1983-11-12)
- DATABASE WPI Section Ch, Week 199720 Derwent Publications Ltd., London, GB; Class B04, AN 1997-213455 XP002189941 & CN 1 097 618 A (LIU J), 25 janvier 1995 (1995-01-25)
- DATABASE WPI Section Ch, Week 198625 Derwent Publications Ltd., London, GB; Class D13, AN 1986-159397 XP002189942 -& JP 61 092530 A (UJI KADOTAEN KK), 10 mai 1986 (1986-05-10)

## Description

L'invention concerne un procédé d'obtention d'une préparation à base de cactus (cactaceae), apte à fixer les graisses et destinée à être ingérée dans le cadre d'un régime alimentaire amincissant par exemple. L'invention concerne également une préparation obtenue par ce procédé, et ses applications.

Le chitosan, dérivé de la chitine contenue notamment dans les parois cellulaires de certains végétaux, bactéries ou crustacés, est un produit non assimilable possédant la propriété de fixer les graisses. Il est utilisé chez l'homme par ingestion, en accompagnement de régimes alimentaires amincissants, pour permettre de soustraire à la digestion les graisses ingérées. Malgré une efficacité reconnue, ce produit est aujourd'hui controversé.

Il est par conséquent souhaitable de trouver de nouveaux produits présentant des propriétés identiques sinon meilleures et répondant aux exigences des experts nutritionnistes.

Les cladodes de certains cactus sont consommés par certaines populations d'Amérique du Sud et d'Amérique Centrale en tant que légumes cuits ou crus en salades. Ces denrées alimentaires sont entre autres appréciées pour leur apport en eau et hydrates de carbone ; elles sont également consommées en tant que plantes médicinales diurétiques, laxatives, antispasmodiques ou encore pour le traitement des fièvres, des ulcères ou de l'hyperglycémie. En particulier, l'Opuntia Ficus Indica -communément appelé Nopal- cultivé au Mexique, est consommé dans le cadre de régimes alimentaires pour le traitement de l'obésité, de l'hyperlipidémie et du diabète. Des études [voir "Effects of Nopal (Opuntia) on serum lipids, glycemia and body weight", archives mexicaines de la recherche médicale, Vol 14 n°2, 1983] menées avec des personnes obèses, diabétiques et saines ont montré que l'ingestion de cladodes de Nopal pendant plusieurs jours avant les trois principaux repas permettait de réduire de façon significative leur taux de LDL ("Low Density Lipoprotein", dit "mauvais cholestérol"), de réduire également le taux de triglycérides et le poids des personnes obèses et diabétiques, ainsi que la glycémie des diabétiques (on constate par ailleurs un étalement dans le temps de l'assimilation des sucres). C'est pourquoi les cladodes de cactus entrent dans la composition de certains produits thérapeutiques, destinés aux dabétiques (voir XP-002189940, Zhejiang prov. people hospital) ou prescrits en cas d'obésité, de "mauvais cholestérol" ou dé troubles digestifs (voir XP-002189939, Iwasaki S).

Toutefois, des tests in vitro effectués par les inventeurs ("test primaire de fixation" décrit plus loin) ont montré que la capacité à fixer les graisses par les cladodes de Nopal tels qu'ils sont vendus dans le commerce pour être consommés est notablement plus faible que celle du chitosan.

L'invention vise à proposer un produit, et un procédé d'obtention d'un tel produit, ayant la propriété de fixer les graisses dans des proportions au moins équivalentes à celles du chitosan. Elle vise à proposer un produit pouvant être ingéré sans risque (c'est-à-dire ne présentant aucune nocivité) et ayant la propriété de fixer des graisses in vivo en vue de les soustraire à la digestion.

Un autre objectif de l'invention est d'utiliser des produits naturels tels que les cladodes de cactus, et d'en améliorer de façon spectaculaire les propriétés sans transformation ni ajout chimiques.

L'invention vise par ailleurs à atteindre tous ces objectifs de façon simple et économique.

A cet effet, l'invention vise une préparation ayant la propriété de fixer les graisses, à base de cladodes d'une cactaceae de type comestible et au moins partiellement non assimilable, caractérisée en ce que la cactaceae se présente sous forme d'une poudre de particules dont au moins 70% en poids ont une taille inférieure à 100 µm. Dans toute la suite, par "particules de taille inférieure à une valeur donnée", on entend : particules ayant franchi, lors d'un processus de tamisage, un tamis présentant des mailles carrées de côté égal à ladite valeur. Par ailleurs, on entend par "cactaceae de type comestible", une cactaceae non toxique, c'est-à-dire pouvant être ingérée sans risque par l'homme ou l'animal. Elle peut ou non posséder des propriétés nutritives. Enfin, on entend par "cactaceae de type au moins partiellement non assimilable", une cactaceae dont une partie au moins n'est pas assimilée par le corps humain (ou animal) lors de la digestion et se retrouve dans les fèces. Dans une version préférée de l'invention, au moins 70% en poids des particules de cactaceae ont une taille inférieure à 75 µm.

Le test primaire de fixation décrit plus loin (effectué avec de l'huile d'olive) appliqué à une telle préparation à base de cladodes d'Opuntia Ficus Indica, donne des rapports de fixation (rapport de la masse de graisses fixée sur la masse de produit "actif" mis en oeuvre-en l'occurrence l'Opuntia, ou le chitosan pour les essais de comparaison-) variant entre 7 et 19, analogues (voire supérieurs dans l'exemple de graisses issues de mayonnaise) à ceux du chitosan, et très supérieurs à ceux des produits existants à base de cladodes de cactus (dont les rapports de fixation varient entre 2 et 5). On constate que, dans la gamme de granulométrie testée, le rapport obtenu croît lorsque la granulométrie moyenne de la poudre de cladode de cactus décroît.

Ce résultat remarquable est inattendu pour l'homme du métier. En effet, les cactus sont essentiellement constitués d'eau (qu'ils retiennent) et de fibres, dont la cellulose, l'hémicellulose, et des fibres alimentaires. Les propriétés chimiques des fibres tenant généralement à la longueur de leurs chaînes, il est surprénant que le broyage fin des cladodes de cactus, qui réduit considérablement la longueur des fibres, améliore la propriété de fixation des graisses dans des proportions aussi spectaculaires, au lieu de la dégrader comme on pourrait s'y attendre. Ceci explique sans doute qu'aucune étude n'ait à ce jour été menée concernant l'influence de la granulométrie sur les propriétés des cladodes de cactus, et qu'aucun des documents antérieurs n'apporte d'enseignement quant à la taille des particules de cactus des poudres connues.

Il est à noter, par ailleurs, que l'homme du métier pourrait être amené à penser que la propriété de fixation des graisses des cladodes de cactus pourrait être améliorée par une hydrophobation desdites fibres (consistant à fixer des chaînes grasses sur le cactus par estérification) : il s'avère cependant qu'un tel traitement chimique appliqué aux produits existants à base de cladodes de Nopal ne procure aucun effet notable sur la fixation des graisses.

Les inventeurs ont également montré que la fixation des graisses par la poudre de cladode de cactus selon l'invention, qui est lipophile, n'engendrait aucun risque de déficience en vitamines liposolubles (voir Test 5 ci-après).

La cactaceae est avantageusement de la famille des Opuntioideae, en particulier du genre des Opuntia. De préférence, il s'agit d'un Opuntia Ficus Indica. Il faut souligner que cette espèce est cultivée de façon intensive en Amérique latine (notamment au Mexique) et commercialisée à faible coût. Ses cladodes sont consommés depuis des siècles par lés populations autochtones, et aucune des nombreuses études menées sur cette plante n'a révélé d'éventuels effets secondaires dus à son ingestion.

Les cladodes de cactaceae sont choisis de préférence jeunes, et notamment âgés de moins de 2 ans. De tels cladodes présentent une teneur garantie en fibres alimentaires solubles et insolubles supérieure à 40%, et procurent, une fois préparés selon l'invention, un meilleur résultat quant à la fixation des graisses.

Avantageusement et selon l'invention, les particules de cactaceae se présentent sous forme de granulés enrobés d'un film à libération contrôlée (ciblée et/ou prolongée).

En particulier, les particules de cactaceae se présentent sous forme de granulés enrobés d'un film acido-résistant, et notamment gastro-résistant, et soluble dans le milieu intestinal. On entend par "granulé" un agglomérat de particules, de forme générale plus ou moins sphéroïde. Les tests primaires de fixation in vitro effectués par les inventeurs ont en effet montré que la quantité de graisses fixée par une poudre de cactus selon l'invention après une incubation en milieu neutre ou légèrement basique (cas de l'intestin) était en moyenne deux fois supérieure à celle fixée après une incubation en milieu acide (cas de l'estomac). L'enrobage permet de préserver ladite poudre des sucs gastriques (milieu de pH 1 à 2) et de la libérer dans l'intestin (pH proche de 7), doublant ainsi son efficacité (rapport de fixation moyen de 20).

Avantageusement et selon l'invention, les particules de cactaceae se présentent sous forme de granulés enrobés d'un film à libération prolongée, contrôlée et progressive dans le temps. Un tel enrobage permet une libération progressive de la poudre selon l'invention au cours de la digestion. En variante, les particules de cactaceae sont incorporées dans une matrice insoluble dans l'eau comprenant par exemple une matière grasse, telle que l'acide stéarique, une matière plastique insensible aux sucs digestifs et/ou un sel ou une combinaison de sels peu soluble(s) dans l'eau, tel(s) que le sulfate de calcium ou le phospate de calcium, ce, en vue de la libération prolongée desdites particules de cactaceae notamment dans l'intestin.

Avantageusement et selon l'invention, l'enrobage est à la fois acido-résisiant, soluble dans l'intestin et à libération prolongée (pour une libération contrôlée et lente dans l'intestin). ,

L'invention vise également un procédé d'obtention d'une préparation ayant la propriété de fixer les graisses, à base de cladodes d'une cactaceae de type comestible et au moins partiellement non assimilable, caractérisé en ce que les cladodes de cactaceae sont séchés puis broyés de façon à obtenir une poudre de particules dont au moins 70% en poids ont une taille inférieure à 100 µm (granulométrie contrôlée par tamisage comme expliqué ci-avant). Dans un mode de mise en oeuvre préféré, les cladodes de cactaceae sont broyés en une poudre dont au moins 70% en poids des particules ont une taille inférieure à 75 µm. Avantageusement et selon l'invention, on utilise une cactaceae de la famille des Opuntioideae, notamment du genre des Opuntia, en particulier de l'espèce des Opuntia Ficus Indica. .

Avantageusement et selon l'invention, les cladodes de cactaceae sont tranchés en morceaux préalablement à leur séchage. Les cladodes et/ou morceaux de cladode de cactaceae sont préférentiellement égouttés à l'air libre pendant 12 à 24 heures préalablement à leur séchage. Les cladodes et/ou morceaux de cladode de cactaceae sont ensuite séchés de façon traditionnelle à l'air libre, notamment dans des séchoirs du type séchoirs ouverts à claies. En variante, on peut notamment utiliser, pour le séchage des cladodes et/ou morceaux de cladode de cactaceae, un séchoir statique ou dynamique maintenu à une température comprise entre 40 et 80°C. La température choisie est adaptée pour ne pas provoquer une transformation de la matière par réchauffement de celle-ci. Avantageusement et selon l'invention, les cladodes et/ou morceaux de cladode de cactaceae sont séchés de façon à présenter une siccité (rapport du poids de matière sèche sur le poids total) finale supérieure ou égale à 90%.

Avantageusement et selon l'invention, les particules de cactaceae obtenues suite au broyage des cladodes et/ou morceaux de cladode de cactaceae sont granulées, et les granulés résultants sont enrobés d'un film à la libération contrôlée, notamment acido-résistant et soluble dans le milieu intestinal et/ou à libération prolongée. On entend par "granulation" (ou par "granuler") la réalisation de ponts liants entre les particules de cactus de façon à obtenir des agglomérats de particules, de forme générale plus ou moins sphéroïde.

Dans un mode de mise en oeuvre avantageux, on réalise la granulation des particules de cactaceae en introduisant lesdites particules dans un lit d'air fluidisé présentant une température d'entrée d'air comprise entre 55 et 65°C, et en pulvérisant dans ledit lit d'air fluidisé une solution de granulation à une pression comprise entre 2,5 et 3,5 bars. A titre d'exemple, pour granuler environ 100 kg de poudre de cactus selon l'invention, on pulvérise la solution de granulation avec un débit compris entre 8 et 12 g/min. Le débit est ajusté en fonction de la taille souhaitée des granulés. On utilise préférentiellement une solution aqueuse de granulation comprenant un agent liant ou une combinaison d'agents liants choisi(s) dans le groupe suivant : gélatine, gomme arabique, gomme de xanthane, gomme de guar, gomme de caroube. Il est à noter que la granulation permet uniquement de réduire la surface de poudre à enrober, et donc de diminuer la quantité de solution d'enrobage utilisée, la durée et le coût du procédé. Les ponts liants créés se rompent instantanément lorsque l'enrobage se dissout et que la poudre est libérée en milieu aqueux. La granulométrie de la poudre reste donc inchangée. Les granulés obtenus à l'issue de l'étape de granulation des particules de cactaceae sont préférentiellement séchés dans un lit d'air fluidisé à une température d'entrée d'air comprise entre 45 et 55°C.

Avantageusement et selon l'invention, on réalise l'enrobage des granulés en introduisant lesdits granulés dans un lit d'air fluidisé présentant une température d'entrée d'air comprise entre 45 et 55°C, et en pulvérisant dans ledit lit d'air fluidisé une solution d'enrobage à une pression comprise entre 2,5 et 3,5 bars, et selon un débit compris entre 6 et 10 g/min si l'on souhaite enrober environ 100 kg de granulés.

Pour réaliser un enrobage acido-résistant et soluble dans l'intestin, on utilise préférentiellement une solution d'enrobage comprenant un solvant tel que l'éthanol, et un agent filmogène ou une combinaison d'agents filmogènes choisi(s) dans le groupe suivant : cellulose acétophtalate, carboxyméthyl-cellulose (CMC). La solution d'enrobage comprend également de préférence un agent plastifiant ou une combinaison d'agents plastifiants choisi(s) dans le groupe suivant : triacétine, polyéthylèneglycol (PEG). Le film résultant est résistant à pH acide et soluble à pH neutre ou légèrement basique. La poudre de cactus ingérée sous forme de granulés enrobés se libère donc au moment où les granulés transitent par l'intestin.

Pour réaliser un enrobage à libération prolongée, on utilise préférentiellement une solution d'enrobage comprenant un agent filmogène ou une combinaison d'agents filmogènes à solubilisation lente dans l'eau, choisi(s) dans le groupe suivant : acétate de polyvinyle, gomme laque ou shellac. En variante, on utilise une solution d'enrobage comprenant un agent filmogène ou une combinaison d'agents filmogènes insoluble(s) dans l'eau mais semi-perméable(s), tel(s) qu'un polymère du type gomme arabique. Ces agents insolubles (mais semi-perméables) ou peu solubles dans l'eau peuvent être combinés à des agents filmogènes acido-résistants et solubles dans l'intestin, en vue de permettre une libération prolongée de la poudre de cactus dans l'intestin uniquement.

En variante, les particules de cactaceae obtenues suite au broyage des cladodes et/ou morceaux de cladode de cactaceae sont incorporées dans une matrice insoluble en vue de permettre une libération prolongée desdites particules lorsqu'elles sont ingérées. Une telle matrice insoluble comprend par exemple une matière grasse, telle que l'acide stéarique, et une matière plastique insensible aux sucs digestifs et/ou un sel peu soluble dans l'eau, tel que le sulfate de calcium ou le phosphate de calcium.

L'invention s'étend à une préparation et à un procédé d'obtention d'une telle préparation, caractérisés en combinaison par tout ou partie des caractéristiques mentionnées ci-dessus et ci-après.

L'invention s'étend également à l'utilisation de cladodes de cactaceae séchés et broyés en une poudre de particules dont au moins 70% en poids ont une taille inférieure à 100 µm, pour l'obtention d'une préparation destinée à être ingérée en association avec une ration de denrées alimentaires en vue de fixer in vivo les graisses présentes dans ladite ration alimentaire.

En particulier, l'invention concerne l'utilisation de cladodes de cactaceae séchés et broyés en une poudre de particules dont au moins 70% en poids ont une taille inférieure à 100 µm, pour l'obtention d'une composition thérapeutique destinée à traiter l'obésité. L'invention concerne également une méthode pour fixer in vivo les graisses et notamment pour traiter l'obésité, selon laquelle on administre au patient par voie orale une préparation à base de cactaceae telle que revendiquée.

L'invention s'étend également à une composition thérapeutique comprenant une préparation à base de cactaceae telle que revendiquée, et un excipient pharmaceutiquement acceptable. Une telle composition thérapeutique est avantageusement conditionnée sous une forme galénique adaptée à une administration par voie orale. A noter que la composition thérapeutique peut contenir d'autres principes actifs compatibles avec la préparation à base de cactaceae selon l'invention.

La poudre de cladode de cactaceae peut être conditionnée seule, sous forme de comprimés, gélules, capsules souples ou rigides ou tout simplement de poudre à mélanger à une boisson (eau ou autre soda, jus de fruit, etc.) ou à saupoudrer sur un aliment. Elle est alors de préférence ingérée au cours d'un repas. En variante, elle peut être intégrée à une denrée alimentaire.

Mélangée à une denrée alimentaire non grasse, elle permet de fixer les graisses des denrées alimentaires grasses ingérées lors d'un même repas. Il est à noter que les cladodes de cactus destinés à être mélangés à un liquide (soda, etc.) sont préférentiellement broyés à une granulométrie inférieure à 20 µm. Mélangée à une denrée alimentaire grasse, la poudre permet d'en fixer les graisses avant (in vitro) et/ou après (in vivo) ingestion de ladite denrée. La poudre doit, dans ce cas, présenter une granulométrie adaptée pour que les particules de cactus et les éventuelles graisses fixées sur ces particules ne soient pas perceptibles dans la bouche lors de la consommation de la denrée alimentaire.

L'invention s'étend aussi à une composition alimentaire - aliment simple ou composé- et/ou à un complément alimentaire comprenant une préparation à base de cactaceae telle que revendiquée. On entend par compléments alimentaires des sources concentrées de nutriments ou d'autres substances ayant un rôle nutritionnel ou physiologique, seuls ou en combinaison, commercialisés sous forme de dose unitaire et dont l'objet est de compléter l'alimentation normale. Les compléments alimentaires répondent à des règles législatives strictes en termes de composition, dosage, étiquetage, etc.. L'invention concerne également l'utilisation d'une préparation à base de cactaceae telle que revendiquée pour l'obtention d'une composition alimentaire et/ou d'un complément alimentaire.

La description qui suit fournit quelques exemples non limitatifs de réalisation selon l'invention d'une préparation à base de cactaceae, ainsi qu'un résumé des études d'efficacité pratiquées sur divers produits dont des préparations selon l'invention.

### TEST 1 :

La propension à fixer les graisses d'une poudre de cladode de cactus a été vérifiée à l'aide d'un dispositif simulant le fonctionnement de l'appareil digestif humain, afin de pouvoir étendre les résultats des tests primaires de fixation au cas particulier de la digestion. Le dispositif utilisé est décrit par US-5.525.305. Il reproduit les conditions dynamiques successives de l'appareil digestif, telles que les variations de pH entre l'estomac et l'intestin grêle, les concentrations de (pro)enzymes, sucs, sels et autres sécrétions des différents organes concernés (suc gastrique avec enzymes, électrolytes, bile, pancréatine, etc.), et les cinétiques de passage du chyme dans l'estomac et les intestins. Ce dispositif permet d'étudier la digestibilité et la disponibilité à l'assimilation des nutriments, ainsi que la stabilité d'ingrédients spécifiques (protéines actives, peptides, etc.). Ce dispositif a été validé par des expériences in vivo.

2 g d'Opuntia Ficus Indica sont mélangés, de façon homogène, à un plat composé de 20 g d'huile de tournesol et 144 g de yaourt écrémé. Une préparation témoin identique (huile + yaourt) mais ne comprenant pas d'Opuntia est également élaborée et ingérée dans le dispositif de simulation lors d'une expérience de contrôle ultérieure. Le dispositif de simulation est paramétré pour reproduire l'appareil digestif d'un adulte jeune et en bonne santé, après ingestion d'une nourriture semi-liquide. Les aliments ingérés séjournent 4 heures dans le dispositif de simulation. Un système spécifique est par ailleurs utilisé pour isoler du dispositif les produits digérés et assimilés.

Sont recueillis et analysés : les matières résiduelles dans l'estomac et le duodénum (tels que simulés par le dispositif) à la fin de l'expérience, les matières résiduelles dans l'intestin grêle à la fin de l'expérience, des échantillons des fractions assimilées à l'issue de chacune des 4 heures d'expérience (lesdits échantillons sont par ailleurs mélangés entre eux, pour partie, pour former un échantillon représentant une fraction de la totalité des aliments assimilés). Les volumes des différents échantillons prélevés sont mesurés, et conservés pour moitié à -20°C pour une double analyse.

La composition des acides gras dans les fractions assimilées est analysée selon le procédé suivant. Les échantillons sont hydrolysés en milieu alcalin pendant une heure à 70°C. Après refroidissement et acidification, un étalon interne est ajouté et les acides gras sont extraits au moyen d'un bain d'hexane. Après évaporation de la phase d'hexane, des esters de méthyle sont préparés à partir desdits acides gras en utilisant un réactif BF₃ méthanol. Après refroidissement, une solution d'hexane et de chlorure de sodium saturée est ajoutée. Après agitation, la phase supérieure d'hexane est prélevée, diluée et soumise à une analyse chromatographique au gaz.

Les résultats sont consignés dans le tableau 1. A noter que la quantité de triglycérides présente initialement dans chacune des préparations nutritives ingérées (préparation témoin et préparation avec Opuntia) est de 985,5 mg/g d'huile, soit au total 19,7 g de triglycérides ingérées par préparation nutritive.

**Tableau 1**

| | Préparation témoin | Préparation avec Opuntia |
|---|---|---|
| Quantité totale d'acides gras assimilée par l'appareil digestif : | 9,40 ± 0,34 g (1) | 6,74 ± 0,38 g (2) |
| Pourcentage d'acides gras assimilés : | 90 ± 9 % | 71 ± 7 % |
| Quantité totale d'acides gras fixée par l'Opuntia (3) = (1) - (2) | 2,64 ± 0.36 g (3) | |
| Rapport de fixation des acides gras ^{*} : | 1,32 ± 0,18 | |

| | | |
|---|---|---|
| ^{*} Dans cet exemple, le rapport de fixation est égal au poids d'acides gras (et non d'huile) fixé par l'Opuntia sur le poids d'Opuntia mis en oeuvre. | | |

L'aptitude de la poudre de cactus (en l'occurrence l'Opuntia Ficus Indica) à fixer les graisses est donc vérifiée dans le cas particulier (du point de vue chimique et cinétique) de la digestion.

La répartition des différents acides gras assimilés est donnée par le tableau 2 pour les deux préparations (témoin et avec Opuntia).

**Tableau 2**

| Acides Gras assimilés | Préparation témoin (mg) | Préparation avec Opuntia (mg) |
|---|---|---|
| C14 | 14,0 ± 4,1 | 9,6 ± 0,09 |
| C16 | 679,8 ± 43,0 | 496,1 ± 38,9 |
| C18 | 354,8 ± 6,6 | 260,2 ± 7,3 |
| C18:1 c | 2210,3 ±120,8 | 1565,9 ± 67,9 |
| C18:2 c | 5368,0 ± 233,8 | 3787,0 ± 158,8 |
| C20:4 c | 94,0 ± 14,4 | 72,2 ± 13,8 |
| Non identifié | 676,3 ± 79,9 | 548,6 ± 91,5 |
| Total | 9397,2 ± 342,0 | 6739,6 ± 378,0 |

On constate que le rapport entre la quantité d'acides gras assimilée dans le cadre de l'expérience témoin et celle assimilée dans le cadre de l'expérience avec Opuntia varie peu en fonction de l'acide gras. La fixation des acides gras par la poudre de cactus n'est donc pas sélective.

### Mode opératoire du test primaire de fixation

### Matériel :

Etuve réglée à 37°C
Balance à 1 mg
Becher 250 mℓ
Tubes bouchés à centrifuger en verre de 85 mℓ (dimensions 44x98 mm)
Micropipettes 100 µℓ et 1000 µℓ
Centrifugeuse réglée à 2000 tours/min (670 g)

### Réactifs :

Acide chlorhydrique 0,1N

| | | | |
|---|---|---|---|
| Tampon : | acide tris(hydroxyméthyl)aminométhane | | 120 g |
| | HCl 35 % | | 49 mℓ |
| | eau | quantité suffisante pour | 200 mℓ |

Trois tubes à essais contenant 25 mℓ d'HCl 0,1N sont préparés. 6 g (masse M) d'huile sont ajoutés dans le premier tube, 12 g (M) de beurre fondu dans le deuxième, et 12 g (M) de mayonnaise dans le troisième. 0,6 g (masse M1) de la préparation à tester (préparation de cactus disponible dans le commerce, poudre de cactus selon l'invention, chitosan...) sont ajoutés dans chacun des tubes. Les solutions obtenues sont mélangées vivement en agitant le tube à la main pendant 30 s, et placées dans une étuve à 37°C pour une incubation de 2 heures. 5 mℓ d'une solution tampon de pH 7,3 sont ensuite ajoutés dans chacun des tubes. Après une nouvelle agitation manuelle, les tubes sont remis dans l'étuve à 37°C pendant 3 heures. Ils sont ensuite centrifugés pendant 5 minutes à 2000 tours/min. La matière grasse surnageante est ensuite prélevée et pesée (masse M2), et le rapport de la masse de corps gras (huile, mayonnaise ou beurre) fixée par la préparation (M-M2) sur la masse de préparation mis en oeuvre (M), dit rapport de fixation, est calculé. Pour chaque préparation testée, le test est répété plusieurs fois pour s'assurer de la validité des résultats.

### TEST 2 : Comparaison des capacités de fixation de quelques produits commerciaux à base de cactus, de trois préparations selon l'invention et de produits commerciaux à base de chitosan

Différents produits disponibles dans le commerce, ainsi que trois préparations selon l'invention ont été testés selon le protocole du test primaire de fixation. Les résultats sont consignés dans le tableau 3 ci-dessous.

**Tableau 3**

| | | Rapport de fixation^{**} | | |
|---|---|---|---|---|
| | Fraction < 75^{*} | huile | mayonnaise | beurre |
| Préparations de cactus disponibles dans le commerce : | | | | |
| 1- Poudre de Nopal, complément des régimes pour diabétiques agit notamment sur la glycémie) - PROESA Std | 19 % | 2 ± 2 | 8 ± 3 | 5 ± 2 |
| | | | | |
| 2- Poudre de Nopal pour diabétiques - PLANTA MEX 60 M | 48% | 1 ± 2 | 13 ± 3 | 4 ± 2 |
| 3- Poudre de Nopal selon l'invention (lot ABBN0075) | 87 % | 8 ± 2 | 20 ± 3 | 10 ± 2 |
| 4- Poudre de Nopal selon l'invention (lot BER001) | 80% | 7 ± 2 | 17 ± 3 | 11 ± 2 |
| 5- Poudre de Nopal selon l'invention (lot SM6041) | 75% | 10 ± 2 | 17 ± 3 | 11 ± 2 |
| 6- BioCaptol®, chitosan, complément des régimes amincissants pour fixer les graisses | nd | 6 ± 2 | 10 ± 3 | 8 ± 2 |
| | | | | |
| 7- UltimateNutrition®, chitosan, pour fixer les graisses | nd | 9 ± 2 | 13 ± 3 | 11 ± 2 |
| | | | | |
| 8- Absorbitol®, chitosan, pour fixer les graisses | nd | 9 ± 2 | 12 ± 3 | 10 ± 2 |

| | | | | |
|---|---|---|---|---|
| ^{*} Fraction < 75 : fraction de particules de taille inférieure à 75 µm, contrôlée par tamisage. ^{**} Rapport de fixation = g d'huile ou de mayonnaise ou de beurre fixés / g de poudre de cactus ou g de chitosan. | | | | |

Les poudres préparées selon l'invention (échantillons 3 à 5) présentent donc des rapports de fixation considérablement supérieurs à ceux des poudres de Nopal commerciales. Le procédé de préparation de ces poudres est donc bien en relation directe avec la capacité du produit à fixer les graisses. Par ailleurs, les poudres selon l'invention ont une efficacité comparable voire significativement supérieure à celle des produits à base de chitosan (échantillons 6 à 8).

### TEST 3 : Influence de la granulométrie

On réalise un profil granulométrique d'une poudre de cladodes d'Opuntia Ficus Indica broyés finement (selon l'invention). Ce profil est réalisé selon la norme NF ISO 2591-1 (tamisage de contrôle) en superposant quatre tamis de 150, 106, 75 et 53 µm d'ouverture de maille (mailles carrées). Les résultats du tamisage sont consignés dans le tableau 4. Chacune des fractions est recueillie en vue de tester sa capacité à fixer les graisses.

**Tableau 4**

| Dimensions des particules d (µm) | Refus sur chaque tamis | | Ouverture nominale de maille (µm) | Passants cumulés (%) |
|---|---|---|---|---|
| | grammes | % | | |
| d>150 | 2,57 | 10,3 | 150 | 89,7 |
| 150 >= d > 106 | 1,62 | 6,5 | 106 | 83,2 |
| 106 >= d > 75 | 1,92 | 7,7 | 75 | 75,5 |
| 75>=d>53 | 2,44 | 9,8 | 53 | 65,6 |
| 53 >= d | 16,34 | 65,6 | 0 | 0 |
| Total | 24,89 | 100 | | |

On mesure ensuite la capacité à fixer l'huile, la mayonnaise et le beurre (test primaire de fixation) de la poudre non tamisée et de chacune des fractions précédentes isolées. Les résultats sont consignés dans le tableau 5.

**Tableau 5**

| | Rapport de fixation : | | |
|---|---|---|---|
| Fraction d'Opuntia testée : | Huile | Mayonnaise | Beurre |
| poudre non tamisée | 10 ± 2 | 19 ± 3 | 12 ± 2 |
| d > 150 µm | 4 ± 2 | 12 ± 3 | 8 ± 2 |
| 150 µm > = d > 106 µm | 5 ± 2 | 13 ± 3 | 8 ± 2 |
| 106 µm > = d > 75 µm | 5 ± 2 | 1.3 ± 3 | 9 ± 2 |
| 75 µm > = d > 53 µm | 7 ± 2 | 15 ± 3 | 13 ± 2 |
| d=<53µm | 10 ± 2 | 19 ± 3 | 13 ± 2 |

Les échantillons précédents provenant d'une même poudre, on peut conclure avec certitude du test 3 que la capacité à fixer l'huile d'une poudre de cactus est une fonction décroissante de la taille des particules (dans la plage de granulométrie testée).

### TEST 4 : Influence du pH

Ce test a pour objectif de comparer la capacité à fixer les graisses d'une même poudre d'Opuntia Ficus Indica, préparée selon l'invention, en fonction du pH du milieu d'incubation. Un premier essai est réalisé dans les conditions du test primaire de fixation déjà décrit, avec 0,3 g de prise d'essai d'échantillon. Un second essai est réalisé en remplaçant les 25 mℓ d'HCl 0,1N de l'essai précédent par 25 mℓ d'eau tamponnée à pH 7 ± 0,1. Les résultats sont consignés dans le tableau 6.

**Tableau 6**

| Protocole de test | Rapport de fixation : |
|---|---|
| Test primaire de fixation avec incubation à pH gastrique (HCl) | 11 ± 2 |
| Test primaire modifié avec incubation à pH intestinal (H₂O) | 20 ± 2 |

On constate un doublement de la capacité de la poudre d'Opuntia à fixer les graisses si l'incubation a lieu dans un milieu neutre, tel que l'intestin. L'incubation à pH gastrique limite donc fortement l'efficacité de la poudre de cactus. C'est pourquoi l'invention prévoit avantageusement de protéger la poudre de cactus par un enrobage acido-résistant et à libération intestinale.

### TEST 5 : Fixation des vitamines liposolubles par une poudre de cactus selon l'invention

Le test consiste à placer un fixateur de graisse en présence d'un corps gras (huile de tournesol) contenant environ l'Apport Nutritionnel Conseillé (ANC) journalier en vitamines A et E, dans des conditions (phase aqueuse, température, pH) similaires à celles de la digestion. Le fixateur de graisse forme un gel avec la matière grasse et diminue la quantité de graisse surnageant dans le milieu. L'analyse de la quantité de vitamines de la phase grasse surnageant sans et avec fixateur de graisse permet d'évaluer la capacité de ce dernier à fixer spécifiquement les vitamines liposolubles.

### Matériel :

Etuve réglée à 37°C
Balance à 1 mg
Verrerie courante de laboratoire
Agitateur magnétique chauffant
Tubes bouchés à centrifuger en verre de 85 mℓ (dimensions 44x98 mm)
Micropipettes 100µℓ et 1000µℓ
Centrifugeuse réglée à 2000 tours/min (soit 670g)

### Réactifs :

Vitamines A et E (fournies par Sigma-Aldrich-Fluka Produits Chimiques)
Acide chlorhydrique 0,1N
Huile de tournesol du commerce (avec teneur en vitamine E garantie)

| | | | |
|---|---|---|---|
| Tampon pH 7,3 : | Acide tris(hydroxyméthyl)aminométhane | | 121,0 g |
| | HCl 35% | | 49,0 mℓ |
| | eau | quantité suffisante pour | 200 mℓ |

On ajoute à l'huile de tournesol du commerce des quantités de vitamines A et E telles que 20g d'huile ainsi améliorée, dite huile standardisée, contiennent approximativement l'ANC pour un adulte de sexe féminin (soit vitamine A : 800 µg, vitamine E : 12 mg).

Dans chacun des deux tubes à centrifuger, on verse 25 mℓ d'HCL 0,1N auxquels on ajoute 20 g d'huile standardisée et 0,6 g de poudre d'Opuntia selon l'invention. Chaque tube bouché est agité énergiquement durant 30 secondes puis mis à incuber pendant 2 heures dans l'étuve à 37°C. On ajoute 5 mℓ de solution tampon pH 7,3 dans chacun des tubes, que l'on rebouche, agite énergiquement durant 30 secondes et remet à incuber dans l'étuve à 37° durant 3 heures. On fait subir à chaque tube une centrifugation à 2000 tours/min durant 5 minutes. Les tubes sont ensuite maintenus à 37°C, pendant que l'on prélève la matière grasse surnageante. La teneur en vitamine A et E du surnageant prélevé est analysée.

On réitère l'expérience précédente en remplaçant la masse d'Opuntia par 0,6 g d'eau distillée, en vue de réaliser un témoin. On compare les teneurs en vitamines A et E dans la matière grasse surnageant en fin d'expérience dans l'essai (en présence de poudre d'Opuntia selon l'invention) et dans le témoin (eau distillée). Les résultats sont reportés dans le tableau 7 ci-dessous.

**Tableau 7**

| | Concentration en vitamines liposolubles de l'huile surnageant en fin d'expérience (pour 100g d'huile) | | Capacité (C)* de fixation spécifique des vitamines par la poudre selon l'invention |
|---|---|---|---|
| | Témoin (V_{témoin}) | Essai (Vₑₛₛₐᵢ) | |
| Vitamine A | 4,65 mg/100g | 4,40 mg/100g | 5,4% |
| (rétinol) | | | |
| Vitamine E | 58,6 mg/100g | 57,5 mg/100g | 1,9% |
| (di-α-tocophérol) | | | |

| | | | |
|---|---|---|---|
| * Capacité de fixation spécifique des vitamines par la poudre d'Opuntia selon l'invention : C = 1- (Vₑₛₛₐᵢ / V_{témoin}) | | | |

Les écarts de concentration observés entre le témoin et l'essai sont faibles. Et il faudrait réaliser une étude statistique pour déterminer s'ils sont effectivement dus à la poudre d'Opuntia selon l'invention, ou s'ils résultent simplement de la manipulation. En tout état de cause, les écarts constatés ne sont pas significatifs et prouvent que la poudre d'Opuntia selon l'invention ne fixe pas les vitamines liposolubles A et E de manière spécifique.

### EXEMPLE 6 : Enrobage

Une poudre selon l'invention est granulée dans un lit d'air fluidisé paramétré comme suit.
Température d'entrée d'air : 60°C
Débit de vaporisation : 10 g/min
Pression d'atomisation : 3 bars
Solution de granulation :
600 mℓ d'H₂O
40g de gélatine 30 Bloom

La poudre d'Opuntia est préalablement chauffée de façon à atteindre une température de 35°C. La solution de granulation est alors pulvérisée sur la poudre en mouvement dans le lit d'air, préférentiellement à l'opposé du distributeur d'air (pulvérisation en "top spray"), ou en partie médiane du dispositif, perpendiculairement au lit d'air. La composition de la solution de granulation et la quantité vaporisée sont ajustées en fonction de la granulométrie finale souhaitée, qui dépend notamment du conditionnement final de la poudre de cactus (comprimés, gélules, capsules rigides ou souples, poudre alimentaire, etc.).

Une fois la granulométrie souhaitée atteinte, la poudre est séchée dans le lit d'air fluidisé dont la température est ajustée à 50°C. Les granulés recueillis sont ensuite tamisés, les granulés de taille trop importante étant broyés à nouveau et réintroduits dans le procédé, les granulés de taille insuffisante étant recyclés lors d'une autre étape de granulation constructive.

Les granulés sont ensuite enrobés dans le lit d'air fluidisé paramétré comme suit.
Température d'entrée d'air : 50°C
Débit de vaporisation : 8 g/min
Pression d'atomisation : 3 bars
Solution d'enrobage :
500 g de cellulose acétophtalate
150 g de triacétine
2ℓ d'éthanol
8ℓ de chlorure de méthylène

Les granulés sont chauffés de façon à atteindre une température de 35°C. La solution d'enrobage est pulvérisée dans le lit d'air fluidisé (en "top spray" par exemple) dans les conditions susmentionnées. Après enrobage, les granulés sont séchés dans le lit d'air fluidisé à une température de 50°C.

### TEST 7 : Propriétés de l'enrobage de l'exemple 6

L'enrobage est tout d'abord testé en milieu acide selon le protocole suivant. On prépare une solution comprenant :
52 ml d'HCl à 37%,
120g de tampon Tris hydroxyméthyl aminométhane
quantité suffisante d'eau pour 200ml de solution,
à laquelle on ajoute 2 g de poudre de Nopal préalablement mélangée au colorant E131 de couleur verte, et enrobée selon l'exemple 6. Le colorant permet de contrôler la libération du Nopal dans le milieu. Après agitation manuelle de la solution, on en effectue une lecture à 420 nm à l'aide d'un spectrophotomètre. Aucune coloration n'apparaît dans la solution : l'enrobage réalisé est résistant à pH très acide (pH approximatif de la solution : 2-3) ; il est donc acido-résistant.

On prépare une solution témoin comprenant les composés susmentionnés et à laquelle on ajoute 2 g de poudre de Nopal préalablement mélangée au colorant E131 mais non enrobée. Selon les résultats du spectrophotomètre, le colorant s'est propagé dans la solution.

L'enrobage est ensuite testé en milieu basique. On prépare à cet effet une solution comprenant:
46 mℓ d'HCl à 37%,
120 g de tampon Tris hydroxyméthyl aminométhane,
la quantité d'eau suffisante pour 200 mℓ de solution,
à laquelle on ajoute 2 g de poudre de Nopal préalablement mélangée au colorant E131 de couleur verte, et enrobée selon l'exemple 6. Après agitation de la solution, on en effectue une lecture à 420 nm à l'aide d'un spectrophotomètre. On détecte un relargage du colorant vert dans le milieu : l'enrobage réalisé est soluble à pH basique (pH approximatif de la solution : 8-9) ; il se libère donc dans l'intestin.

## Revendications

1. - Préparation ayant la propriété de fixer les graisses, à base de cladodes d'une cactaceae de type comestible et au moins partiellement non assimilable, **caractérisée en ce que** la cactaceae se présente sous forme d'une poudre de particules dont au moins 70% en poids ont une taille inférieure à 100 µm.

2. - Préparation selon la revendication 1, **caractérisée en ce qu'**au moins 70% en poids des particules de cactaceae ont une taille inférieure à 75 µm.

3. - Préparation selon l'une des revendications 1 ou 2, **caractérisée en que** la cactaceae est de la famille des Opuntioideae, en particulier du genre des Opuntia, notamment de l'espèce des Opuntia Ficus Indica.

4. - Préparation selon l'une des revendications 1, 2 ou 3, **caractérisée en ce que** les cladodes de cactaceae sont âgés de moins de 2 ans.

5. - Préparation selon l'une des revendications 1 à 4, **caractérisée en ce que** les particules de cactaceae se présentent sous forme de granulés enrobés d'un film acido-résistant et soluble dans le milieu intestinal.

6. - Préparation selon l'une des revendications 1 à 5, **caractérisée en ce que** les particules de cactaceae se présentent sous forme de granulés enrobés d'un film à libération prolongée.

7. - Préparation selon l'une des revendications 1 à 5, **caractérisée en ce que** les particules de cactaceae sont incorporées dans une matrice insoluble dans l'eau comprenant une matière grasse, telle que l'acide stéarique, une matière plastique insensible aux sucs digestifs et/ou un sel, ou une combinaison de sels, peu soluble(s) dans l'eau, tel(s) que le sulfate de calcium ou le phosphate de calcium.

8. - Procédé d'obtention d'une préparation ayant la propriété de fixer les graisses, à base de cladodes d'une cactaceae de type comestible et au moins partiellement non assimilable, **caractérisé en ce que** les cladodes de cactaceae sont séchés puis broyés de façon à obtenir une poudre de particules dont au moins 70% en poids ont une taille inférieure à 100 µm.

9. - Procédé selon la revendication 8, **caractérisé en ce que** les cladodes de cactaceae sont broyés en une poudre dont au moins 70% en poids des.particules ont une taille inférieure à 75 µm.

10. - Procédé selon l'une des revendications 8 ou 9, **caractérisé en ce que** l'on utilise une cactaceae de la famille des Opuntioideae, en particulier du genre des Opuntia, notamment de l'espèce des Opuntia Ficus Indica.

11. - Procédé selon l'une des revendications 8, 9 ou 10, **caractérisé en ce que** l'on utilise des cladodes de cactaceae âgés de moins de 2 ans.

12. - Procédé selon l'une des revendications 8 à 11, **caractérisé en ce que** les cladodes de cactaceae sont tranchés en morceaux préalablement à leur séchage.

13. - Procédé selon l'une des revendications 8 à 12, **caractérisé en ce que** les cladodes et/ou morceaux de cladode de cactaceae sont égouttés à l'air libre pendant 12 à 24 heures préalablement à leur séchage.

14. - Procédé selon l'une des revendications 8 à 13, **caractérisé en ce que** les cladodes et/ou morceaux de cladode de cactaceae sont séchés de façon traditionnelle à l'air libre.

15. - Procédé selon l'une des revendications 8 à 14, **caractérisé en ce que** l'on utilise, pour le séchage des cladodes et/ou morceaux de cladode de cactaceae, un séchoir statique ou dynamique maintenu à une température comprise entre 40 et 80°C.

16. - Procédé selon l'une des revendications 8 à 15, **caractérisé en ce que** les cladodes et/ou morceaux de cladode de cactaceae sont séchés de façon à présenter une siccité finale supérieure ou égale à 90%.

17. - Procédé selon l'une des revendications 8 à 16, **caractérisé en ce que** les particules de cactaceae obtenues sont incorporées dans une matrice insoluble dans l'eau comprenant une matière grasse, telle que l'acide stéarique, et une matière plastique insensible aux sucs digestifs et/ou un sel, ou une combinaison de sels, peu soluble(s) dans l'eau choisi(s) dans le groupe suivant : sulfate de calcium, phosphate de calcium.

18. - Procédé selon l'une des revendications 8 à 16, **caractérisé en ce que** les particules de cactaceae obtenues sont granulées, et **en ce que** les granulés sont enrobés d'un film acido-résistant et soluble dans l'intestin et/ou à libération prolongée.

19. - Procédé selon la revendication 18, **caractérisé en ce que** l'on réalise la granulation des particules de cactaceae en introduisant lesdites particules dans un lit d'air fluidisé présentant une température d'entrée d'air comprise entre 55 et 65°C, et en pulvérisant dans ledit lit d'air fluidisé une solution de granulation à une pression comprise entre 2,5 et 3,5 bars.

20. - Procédé selon la revendication 19, **caractérisé en ce que** l'on utilise une solution aqueuse de granulation comprenant un agent liant ou une combinaison d'agents liants choisi(s) dans le groupe suivant : gélatine, gomme arabique, gomme de xanthane, gomme de guar, gomme de caroube.

21. - Procédé selon l'une des revendications 18, 19 ou 20, **caractérisé en ce que** les granulés obtenus à l'issue de l'étape de granulation des particules de cactaceae sont séchés dans un lit d'air fluidisé présentant une température d'entrée d'air comprise entre 45 et 55°C.

22. -Procédé selon l'une des revendications 18 à 21, **caractérisé en ce que** l'on réalise l'enrobage des granulés en introduisant lesdits granulés dans un lit d'air fluidisé présentant une température d'entrée d'air comprise entre 45 et 55°C, et en pulvérisant dans ledit lit d'air fluidisé une solution d'enrobage à une pression comprise entre 2,5 et 3,5 bars.

23. - Procédé selon la revendication 22, **caractérisé en ce que** l'on utilise une solution d'enrobage comprenant un agent filmogène ou une combinaison d'agents filmogènes acido-résistants et solubles dans le milieu intestinal, et/ou à solubilisation lente dans l'eau ou insolubles dans l'eau mais semi-perméables, choisi(s) dans le groupe suivant : cellulose acétophtalate, carboxyméthyl-cellulose, acétate de polyvinyle, gomme laque, polymère du type gomme arabique.

24. - Procédé selon la revendication 23, **caractérisé en ce que** l'on utilise une solution d'enrobage comprenant un agent plastifiant ou une combinaison d'agents plastifiants choisi(s) dans le groupe suivant : triacétine, polyéthylèneglycol.

25. - Utilisation de cladodes de cactaceae séchés et broyés en une poudre de particules dont au moins 70% en poids ont une taille inférieure à 100 µm, pour l'obtention d'une préparation destinée à être ingérée en association avec une ration de denrées alimentaires en vue de fixer in vivo les graisses présentes dans ladite ration alimentaire.

26. - Utilisation de cladodes de cactaceae séchés et broyés en une poudre de particules dont au moins 70% en poids ont une taille inférieure à 100 µm, pour l'obtention d'une composition thérapeutique destinée à traiter l'obésité.

27. - Composition thérapeutique **caractérisée en ce qu'**elle comprend une préparation selon l'une des revendications 1 à 7 et un excipient pharmaceutiquement acceptable.

28. - Composition thérapeutique selon la revendication 29, sous une forme galénique adaptée à une administration par voie orale.

29. - Composition alimentaire **caractérisée en ce qu'**elle comprend une préparation selon l'une des revendications 1 à 7.

30. -Complément alimentaire **caractérisé en ce qu'**il comprend une préparation selon l'une des revendications 1 à 7.

## Claims

1. Preparation having the property of fixing fats, based on cladodes of a cactacea plant of edible and at least partially non-assimilable type, **characterized in that** the cactacea plant is in the form of a powder of particles, at least 70% by weight of which are smaller than 100 µm.

2. Preparation according to claim 1, **characterized in that** at least 70% by weight of the cactacea plant particles are smaller than 75 µm.

3. Preparation according to either of claims 1 and 2, **characterized in that** the cactacea plant is of the Opuntioidea family, in particular of the genus Opuntia and especially of the species Opuntia Ficus Indica.

4. Preparation according to one of claims 1, 2 and 3, **characterized in that** the cactacea plant cladodes are less than two years old.

5. Preparation according to one of claims 1 to 4, **characterized in that** the cactacea plant particles are in the form of granules coated with an acid-resistant film that is soluble in the intestinal medium.

6. Preparation according to one of claims 1 to 5, **characterized in that** the cactacea plant particles are in the form of granules coated with a sustained-release film.

7. Preparation according to one of claims 1 to 5, **characterized in that** the cactacea plant particles are incorporated into a water-insoluble matrix comprising a fatty substance, such as stearic acid, a plastic that is insensitive to the digestive juices and/or a salt, or a combination of salts, which is (are) sparingly soluble in water, such as calcium sulfate or calcium phosphate.

8. Process for obtaining a preparation having the property of fixing fats, based on cladodes of a cactacea plant of edible and at least partially non-assimilable type, **characterized in that** the cactacea plant cladodes are dried and then comminuted so as to obtain a powder of particles, at least 70% by weight of which are smaller than 100 µm.

9. Process according to claim 8, **characterized in that** the cactacea plant cladodes are comminuted into a powder, at least 70% by weight of the particles of which are smaller than 75 µm.

10. Process according to either of claims 8 and 9, **characterized in that** a cactacea plant of the family Opuntioidea, in particular of the genus Opuntia and especially of the species Opuntia Ficus Indica, is used.

11. Process according to one of claims 8, 9 and 10, **characterized in that** cactacea plant cladodes less than two years old are used.

12. Process according to one of claims 8 to 11, **characterized in that** the cactacea plant cladodes are sliced into pieces before being dried.

13. Process according to one of claims 8 to 12, **characterized in that** the cactacea plant cladodes and/or cladode pieces are drained in the open air for 12 to 24 hours before being dried.

14. Process according to one of claims 8 to 13, **characterized in that** the cactacea plant cladodes and/or cladode pieces are conventionally dried in the open air.

15. Process according to one of claims 8 to 14, **characterized in that** a static or dynamic dryer maintained at a temperature of between 40 and 80°C is used to dry the cactacea plant cladodes and/or cladode pieces.

16. Process according to one of claims 8 to 15, **characterized in that** the cactacea plant cladodes and/or cladode pieces are dried so as to have a final dryness of greater than or equal to 90%.

17. Process according to one of claims 8 to 16, **characterized in that** the cactacea plant particles obtained are incorporated into a water-insoluble matrix comprising a fatty substance, such as stearic acid, and a plastic that is insensitive to the digestive juices and/or a salt, or a combination of salts, that is (are) sparingly soluble in water, chosen from the following group: calcium sulfate, calcium phosphate.

18. Process according to one of claims 8 to 16, **characterized in that** the cactacea plant particles obtained are granulated, and **in that** the granules are coated with an acid-resistant film that is soluble in the intestine and/or that is a sustained-release film.

19. Process according to claim 18, **characterized in that** the cactacea plant particles are granulated by introducing the said particles into a fluidized-air bed with an air inlet temperature of between 55 and 65°C, and by spraying into the said fluidized-air bed a granulation solution at a pressure of between 2.5 and 3.5 bar.

20. Process according to claim 19, **characterized in that** an aqueous granulation solution is used comprising a binder or a combination of binders chosen from the following group: gelatin, gum arabic, xanthan gum, guar gum, carob gum.

21. Process according to one of claims 18, 19 and 20, **characterized in that** the granules obtained after the step of granulating the cactacea plant particles are dried in a fluidized-air bed with an air inlet temperature of between 45 and 55°C.

22. Process according to one of claims 18 to 21, **characterized in that** the granules are coated by introducing the said granules into a fluidized-air bed with an air inlet temperature of between 45 and 55°C, and by spraying into the said fluidized-air bed a coating solution at a pressure of between 2.5 and 3.5 bar.

23. Process according to claim 22, **characterized in that** a coating solution is used comprising a film-forming agent or a combination of film-forming agents that is (are) acid-resistant and soluble in the intestinal medium, and/or that show slow dissolution in water or are insoluble in water but semi-permeable, chosen from the following group: cellulose acetophthalate, carboxymethylcellulose, polyvinyl acetate, shellac, polymer of the gum arabic type.

24. Process according to claim 23, **characterized in that** a coating solution is used comprising a plasticizer or a combination of plasticizers chosen from the following group: triacetin, polyethylene glycol.

25. Use of cactacea plant cladodes dried and comminuted into a powder of particles, at least 70% by weight of which are smaller than 100 µm, to obtain a preparation intended to be ingested in combination with a food ration in order to fix in vivo the fats present in the said food ration.

26. Use of cactacea plant cladodes dried and comminuted into a powder of particles, at least 70% by weight of which are smaller than 100 µm, to obtain a therapeutic composition for treating obesity.

27. Therapeutic composition, **characterized in that** it comprises a preparation according to one of claims 1 to 7 and a pharmaceutically acceptable excipient.

28. Therapeutic composition according to claim 29, in a presentation form that is suitable for oral administration.

29. Food composition, **characterized in that** it comprises a preparation according to one of claims 1 to 7.

30. Food supplement, **characterized in that** it comprises a preparation according to one of claims 1 to 7.

## Patentansprüche

1. Zubereitung mit fettbindender Eigenschaft auf der Grundlage von Kladodien einer Cactacea der eßbaren Art und mindestens teilweise nicht assimilierbar, **dadurch gekennzeichnet, daß** die Cactacea die Form eines Partikelpulvers hat, von dem mindestens 70 Gewichtsprozent eine Größe von unter 100 µm haben.

2. Zubereitung nach Anspruch 1, **dadurch gekennzeichnet, daß** mindestens 70 Gewichtsprozent der Cactaceaepartikel eine Größe von unter 75 µm haben.

3. Zubereitung nach einem beliebigen der vorstehenden Ansprüche 1 oder 2, **dadurch gekennzeichnet, daß** die Cactacea zur Familie der Opuntioideae und insbesondere zur Art der Opuntia Ficus Indica gehört.

4. Zubereitung nach einem beliebigen der vorstehenden Ansprüche 1, 2 oder 3, **dadurch gekennzeichnet, daß** die Kladodien der Cactaceae unter 2 Jahre alt sind.

5. Zubereitung nach einem beliebigen der vorstehenden Ansprüche 1 bis 4, **dadurch gekennzeichnet, daß** die Cactaceaepartikel die Form von Granulat mit einem säureresistenten Film ummantelt haben und im Darmmilieu löslich sind.

6. Zubereitung nach einem beliebigen der vorstehenden Ansprüche 1 bis 5, **dadurch gekennzeichnet, daß** die Cactaceaepartikel die Form von Granulat haben, das mit einem Film mit langfristiger Freisetzung ummantelt ist.

7. Zubereitung nach einem beliebigen der vorstehenden Ansprüche 1 bis 5, **dadurch gekennzeichnet, daß** die Cactaceaepartikel einer im Wasser unlöslichen Matrix zugesetzt sind, die einen Fettstoff umfaßt, wie zum Beispiel die Stearinsäure, einen gegen die Verdauungssäfte unempfindlichen Kunststoff und/oder ein Salz, oder eine Kombination aus Salzen, das/die wenig wasserlöslich ist/sind, wie zum Beispiel Kalziumsulfat oder Kalziumphosphat.

8. Verfahren zur Erzielung einer Zubereitung mit fettbindender Eigenschaft auf Grundlage von Kladodien einer Cactacea von eßbarer Art und zumindest teilweise nicht assimilierbar, **dadurch gekennzeichnet, daß** die Kladodien der Cactacea getrocknet und dann gemahlen werden, bis ein Partikelpulver entsteht, von dem mindestens 70 Gewichtsprozent eine Größe von unter 100 µm haben.

9. Verfahren nach Anspruch 8, **dadurch gekennzeichnet, daß** die Kladodien von Cactaceae zu einem Pulver gemahlen werden, von dem mindestens 70 Gewichtsprozent eine Größe von unter 75 µm haben.

10. Verfahren nach einem beliebigen der vorstehenden Ansprüche 8 oder 9, **dadurch gekennzeichnet, daß** eine Cactacea aus der Familie der Opuntioideae und insbesondere von der Art der Opuntia Ficus Indica verwendet wird.

11. Verfahren nach einem beliebigen der vorstehenden Ansprüche 8, 9 oder 10, **dadurch gekennzeichnet, daß** Kladodien von Cactaceae verwendet werden, die unter 2 Jahre alt sind.

12. Verfahren nach einem beliebigen der vorstehenden Ansprüche 8 bis 11, **dadurch gekennzeichnet, daß** die Kladodien von Cactaceae vor ihrem Trocknen in Stücke geschnitten werden.

13. Verfahren nach einem beliebigen der vorstehenden Ansprüche 8 bis 12, **dadurch gekennzeichnet, daß** die Kladodien und/oder Stücke von Kladodien von Cactaceae vor ihrem Trocknen 12 bis 24 Stunden lang im Freien abgetropft werden.

14. Verfahren nach einem beliebigen der vorstehenden Ansprüche 8 bis 13, **dadurch gekennzeichnet, daß** die Kladodien und/oder Stücke von Kladodien von Cactaceae auf herkömmliche Weise im Freien getrocknet werden.

15. Verfahren nach einem beliebigen der vorstehenden Ansprüche 8 bis 14, **dadurch gekennzeichnet, daß** zum Trocknen der Kladodien und/oder Stücke von Kladodien von Cactaceae ein statischer oder dynamischer Trockner benutzt wird, der bei einer Temperatur von zwischen 40 und 80°C gehalten wird.

16. Verfahren nach einem beliebigen der vorstehenden Ansprüche 8 bis 15, **dadurch gekennzeichnet, daß** die Kladodien und/oder Stücke von Kladodien von Cactaceae so getrocknet werden, daß sie eine Endtrockenheit von über oder gleich 90% aufweisen.

17. Verfahren nach einem beliebigen der vorstehenden Ansprüche 8 bis 16, **dadurch gekennzeichnet, daß** die erzielten Cactaceaepartikel einer im Wasser unlöslichen Matrix zugesetzt sind, die einen Fettstoff umfaßt, wie zum Beispiel die Stearinsäure, und einen gegen die Verdauungssäfte unempfindlichen Kunststoff und/oder ein Salz, oder eine Kombination aus Salzen, das/die wenig wasserlöslich ist/sind, und das/die aus der folgenden Gruppe gewählt wird/werden: Kalziumsulfat oder Kalziumphosphat.

18. Verfahren nach einem beliebigen der vorstehenden Ansprüche 8 bis 16, **dadurch gekennzeichnet, daß** die erzielten Cactaceaepartikel gekörnt werden, und daß das Granulat mit einem säureresistenten Film ummantelt ist, der im Darm löslich und/oder mit langfristiger Freisetzung ist.

19. Verfahren nach Anspruch 18, **dadurch gekennzeichnet, daß** die Kornbildung der Cactaceaepartikel realisiert wird, indem die besagten Partikel in einen Wirbelschicht-Granulator eingeführt werden, der eine Temperatur am Lufteingang von zwischen 55 und 65°C aufweist, und indem im besagten Wirbelschicht-Granulator eine Körnungslösung unter einem Druck von zwischen 2,5 und 3,5 Bar gesprüht wird.

20. Verfahren nach Anspruch 19, **dadurch gekennzeichnet, daß** eine wäßrige Körnungslösung verwendet wird, die ein Bindemittel oder eine Kombination von Bindemitteln umfaßt, das/die aus der folgenden Gruppe gewählt wird/werden: Gelatine, Gummi arabicum, Xanthangummi, Guargummi, Karubegummi.

21. Verfahren nach einem beliebigen der vorstehenden Ansprüche 18, 19 oder 20, **dadurch gekennzeichnet, daß** das bei der Kornbildungsphase der Cactaceaepartikel erzielte Granulat in einem Wirbelschicht-Granulator getrocknet wird, der eine Temperatur am Lufteintritt von zwischen 45 und 55°C aufweist.

22. Verfahren nach einem beliebigen der vorstehenden Ansprüche 18 bis 21, **dadurch gekennzeichnet, daß** die Ummantelung des Granulats realisiert wird, indem das besagte Granulat in einen Wirbelschicht-Granulator gegeben wird, der eine Temperatur am Lufteintritt von zwischen 45 und 55°C aufweist, und indem im besagten Wirbelschicht-Granulator eine Ummantelungslösung unter einem Druck von zwischen 2,5 und 3,5 Bar zerstäubt wird.

23. Verfahren nach Anspruch 22, **dadurch gekennzeichnet, daß** eine Ummantelungslösung verwendet wird, die einen filmbildenden Stoff oder eine Kombination aus filmbildenden säureresistenten Stoffen enthält, der/die im Darmmilieu löslich ist/sind und/oder im Wasser langsam löslich ist/sind, oder im Wasser unlöslich ist/sind, jedoch halbdurchlässig ist/sind, der/die aus der folgenden Gruppe gewählt wird/werden: Acetophtalat-Zellulose, CarboxymethylZellulose, Polyvinylacetat, Gummilack, Polymer von der Art Gummi arabicum.

24. Verfahren nach Anspruch 23, **dadurch gekennzeichnet, daß** eine Ummantelungslösung verwendet wird, die einen Weichmacher oder eine Kombination aus Weichmachern enthält, der/die aus der folgenden Gruppe gewählt wird/werden: Triacetin, Polyethylenglykol.

25. Einsatz von Kladodien von Cactaceae, die getrocknet und zu einem Partikelpulver gemahlen werden, von dem mindestens 70 Gewichtsprozent eine Größe von unter 100 µm haben, dies für eine Zubereitung, die zusammen mit einer Ration Nahrungsmitteln eingenommen werden soll, um in vivo die Fette zu binden, die in dieser Ration Nahrungsmittel enthalten sind.

26. Einsatz von Kladodien von Cactaceae, die getrocknet und zu einem Partikelpulver gemahlen werden, von dem mindestens 70 Gewichtsprozent eine Größe von unter 100 µm haben, dies zur Erzielung einer therapeutischen Zusammensetzung, mit der die Dickleibigkeit behandelt werden soll.

27. Therapeutische Zusammensetzung, **dadurch gekennzeichnet, daß** sie eine Zubereitung nach einem der Ansprüche 1 bis 7 und eine akzeptierbare Grundmasse hat.

28. Therapeutische Zusammensetzung nach Anspruch 29 in einer galenischen Form, die zur oralen Einnahme verabreicht werden kann.

29. Nahrungsmittel-Zusammensetzung, **dadurch gekennzeichnet, daß** sie eine Zubereitung nach einem der Ansprüche 1 bis 7 enthält.

30. Nahrungsergänzungsmittel, **dadurch gekennzeichnet, daß** es eine Zubereitung nach einem der Ansprüche 1 bis 7 enthält.
